# EUROPÄISCHE PATENTSCHRIFT

(11) **EP 1 972 311 B1**
(45) Veröffentlichungstag und Bekanntmachung des Hinweises auf die Patenterteilung: **20.07.2011**
(21) Anmeldenummer: 08005329.1
(22) Anmeldetag: 20.03.2008
(51) Int. Cl.: A61F 5/56

(54) **Biokompatibles Behandlungsgerät zur Therapie des Schnarchens und schlafbezogener Atemstörungen**
Biocompatible treatment device for providing therapy for snoring and sleep-related breathing disturbances
Appareil de traitement biocompatible destiné au traitement du ronflement et des gênes respiratoires liées au sommeil

(30) Priorität: 20.03.2007 DE 102007013879
(43) Veröffentlichungstag der Anmeldung: 24.09.2008
(73) Patentinhaber: bredent GmbH & Co. KG, 89250 Senden (DE)
(72) Erfinder: Brehm, Peter, 89250 Senden (DE); Weiss, Margit, 89073 Ulm (DE)
(74) Vertreter: Dziewior, Joachim

(56) Entgegenhaltungen:
- DE-A1- 10 331 531
- DE-C1- 10 216 242
- DE-U1- 20 102 432
- DE-U1- 20 319 088
- US-A- 6 109 265
- US-A1- 2003 217 753
- US-B1- 6 418 933

## Beschreibung

Die Erfindung betrifft ein biokompatibles Behandlungsgerät zur Therapie des Schnarchens und schlafbezogener Atemstörungen von Patienten.

Zur Behandlung bzw. Beseitigung derartiger Probleme gibt es aus der Literatur zahlreiche Vorschläge, die bis zu operativen Eingriffen hin reichen. Neben den teilweise bestehenden Risiken bei der Anwendung derartiger Verfahren ist teilweise auch nur ein sehr eingeschränkter Erfolg zu erreichen.

Ein Behandlungsgerät gemäß dem Oberbegriff von Anspruch 1 ist aus der US-A-6 418 933 bekannt.

Der Erfindung liegt die Aufgabe zugrunde, ein Behandlungsgerät der eingangs genannten Art zu schaffen, das sich einerseits durch einfachen Aufbau auszeichnet, für den Patienten bei der Anwendung nur eine geringe Beeinträchtigung darstellt und es im übrigen ermöglicht, nur bedarfsweise zur Anwendung zu kommen.

Ein diese Aufgabe lösendes Behandlungsgerät ist in Anspruch 1 definiert.

Der durch die Erfindung erreichte Vorteil besteht im wesentlichen darin, dass das Behandlungsgerät den Unterkiefer während des Schlafes in Vorschubstellung bei gleichzeitiger Bisshebung hält. Dadurch wird verhindert, dass durch das Abschlaffen der Muskulatur im Mund- und Rachenraum beim Schlafen eine Verengung der oberen Atemwege auftritt, deren Folge bei einem unvollständigen Verschluss das Schnarchen und bei einem vollständigen Verschluss der Luftwege sogar Atemaussetzer sind.

Die Anwendung der Schienen erweitert darüber hinaus den Rachenraum, beseitigt also Atemwegshindernisse und befreit so unmittelbar die Schnarchprobleme.

Gemäß der Erfindung ist das Teleskopsystem von zwei Teleskopelementen gebildet, die jeweils vestibulär angeordnet sind.

Um auf besonders einfache Weise den Unterkiefer in Vorschubstellung zu halten, erstrecken sich die Teleskopelemente zweckmäßigerweise vom unteren Zahnbogen anterior zum oberen Zahnbogen posterior.

Gemäß der Erfindung sind die Teleskopelemente endseitig jeweils mit einem Kugelkopf versehen, da hierdurch eine besonders schlanke und somit für den Patienten weniger hinderliche Bauform erreicht wird.

Um den gewünschten Druck der Teleskopelemente auf die Schienen aufzubringen, sind die Teleskopelemente in ihrem Inneren mit einer Druckfeder versehen.

Weiter hat es sich im Rahmen der Erfindung als vorteilhaft erwiesen, wenn die Teleskopelemente von zwei Teleskophülsen und einer Teleskopstange gebildet sind. Dadurch ist ein relativ weitergehender Hub, also auch eine vollständige Öffnungsstellung des Mundes ermöglicht.

Die den Kugelkopf tragende Teleskopfhülse ist mit einer Innenteleskopschraube versehen, der die Druckfeder anliegt und die über eine Bohrung im Kugelkopf einstellbar ist. Auf diese Weise kann eine Feineinstellung der von der Druckfeder auf das Teleskopsystem abgegebenen Kraft erfolgen.

Hierbei ist es weiter möglich, daß die Druckfeder austauschbar in dem Teleskopelement angeordnet ist.

Die Schienen sind in bevorzugter Ausführungsform der Erfindung aus antiallergenem Kunststoff im Spritzgußverfahren hergestellt.

Zur Herstellung der Schienen bieten sich verschiedene Möglichkeiten ann; bei einer vorteilhaften Ausgestaltung sind die Schienen aus einer Tiefziehfolie hergestellt, die aus einem Folienverbund aus Polyethylenterephthalat, Polyethylen und Polyurethan bestehen.

Es besteht jedoch auch die Möglichkeit, daß die Schienen aus einem Kaltpolymerisat hergestellt sind.

Ferner können die Schienen aus Polymethylmethacrylat, insbesondere in Granulatform hergestellt sein, wobei das Material auf etwa 260° erhitzt und anschließend unter einem Druck von etwa 9 Bar in eine Küvettenform gepreßt wird. In gleichem Sinne ist es zweckmäßig, wenn die Teleskopelemente aus Titan bestehen.

Die Kugelköpfe können in unterschiedlicher Weise, insbesondere auch direkt in der Schiene gelagert sein; vorteilhafterweise werden die Kugelköpfe jedoch in Matrizen gelagert, die in die Schienen einpolymerisiert sind. Hierdurch können die Lagerschalen mit optimalen Lagereigenschaften versehen werden.

Schließlich kann im frontalen Bereich der Schienen eine diese gegenseitig verriegelnde Verschlußeinrichtung vorgesehen sein, wodurch auch im Schlaf eine sichere Abstützung des Kiefers und damit eine Entspannung der Muskulatur erreicht wird.

Hierbei kann die Verschlußeinrichtung als Kugelknopfarretierung ausgebildet sein, wobei eine Ausgestaltung derart möglich ist, daß die Kugelknopfarretierung einen Rastsitz bildet. Dies ergibt eine relativ feste Lagefixierung in einer bevorzugten Ausrichtung der Schienen und damit der Kiefer. Die Kugelknopfarretierung kann aber auch als eine lose Entlastungsposition ausgebildet sein, die unter dem Einfluß der Telekopelemente bevorzugt eingenommen wird, die aber ohne weiteres wieder verlassen werden kann, wenn dies vorübergehend gewünscht ist.

Schließlich können auf wenigstens einer der Schienen Bisswälle zur Abstützung und damit zur Entlastung der Muskulatur, Kiefer und Gelenke vorgesehen sein.

Diese Bisswälle erstrecken sich vorteilhaft von okklusal nach buccal.

Im folgenden wird die Erfindung an einem in der Zeichnung dargestellten Ausführungsbeispiel näher erläutert; es zeigen:
- Fig. 1: das Behandlungsgerät nach der Erfindung, im Einsatz bei einem schlafenden Patienten,

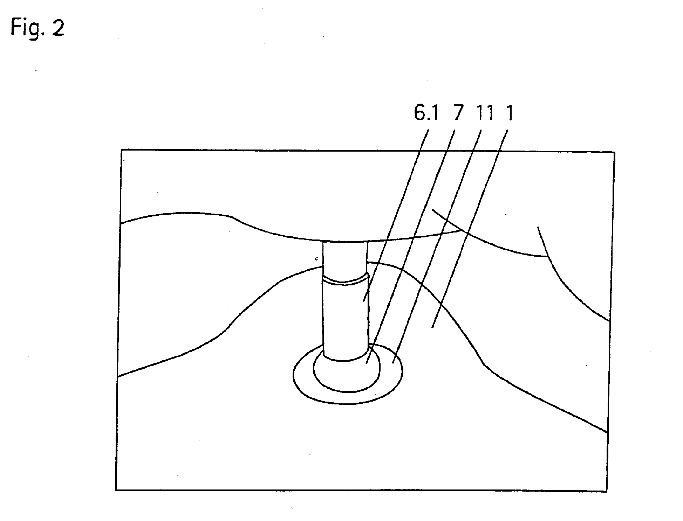

- Fig. 2: eine Schiene des Behandlungsgerätes mit angesetztem Teleskopsystem,
- Fig. 3: ein einzelnes Teleskopelement mit endseitig angebrachten Kugelköpfen,
- Fig. 4: ein Teleskopelement mit zur Einbettung in die Schienen vorgesehenen Matrizen.
- Fig. 5: eine frontal an den Schienen angebrachte Verschlußeinrichtung,
- Fig. 6: eine Seitenansicht der Schienen mit angebrachtem Bisswall.

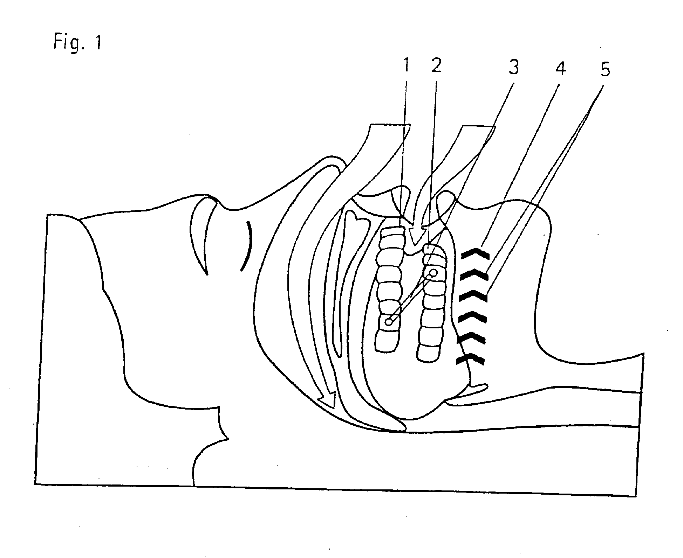

Das in der Zeichnung dargestellte Behandlungsgerät dient zur Therapie des Schnarchens und schlafbezogener Atemstörungen von Patienten. Es besteht, wie aus der Fig. 1 erkennbar, aus einem Doppelschienensystem, das je eine Schiene 1, 2 für den Ober- und den Unterkiefer aufweist. Beide Schienen sind über ein elastisches Teleskopsystem 3 miteinander verbunden, wobei das Teleskopsystem 3 an seinen beiden Enden jeweils gelenkig an eine der beiden Schienen 1, 2 angeschlossen ist. Hierdurch wird erreicht, dass der Unterkiefer 4 während des Schlafes in Vorschubstellung, also in Richtung der Pfeile 5 bei gleichzeitiger Bisshebung gehalten wird. Die zur Anwendung kommende Schiene sorgt darüber hinaus für eine Erweiterung des Rachenraumes, wodurch Atemwegshindernisse beseitigt werden und somit die zu vermeidenden Schnarchprobleme nicht mehr auftreten können.

Das Teleskopsystem 3 wird von zwei Teleskopelementen 6 gebildet, die jeweils vestibulär angeordnet sind. Dabei erstrecken sich die Teleskopelemente 6 vom unteren Zahnbogen anterior zum oberen Zahnbogen posterior.

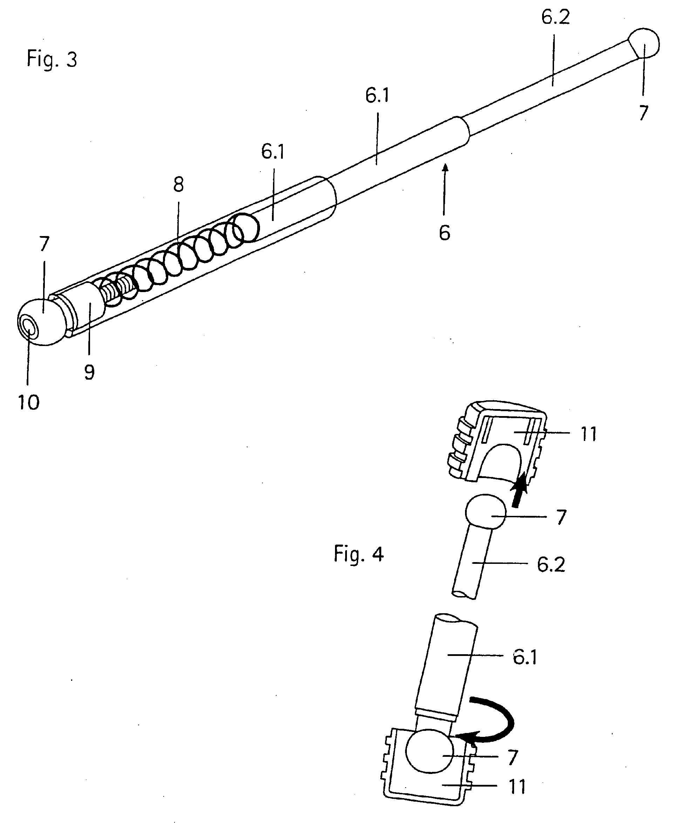

Wie sich insbesondere aus den Fig. 3 und 4 ersehen läßt, sind die Teleskopelemente 6 endseitig jeweils mit einem Kugelkopf 7 versehen, wodurch eine sehr schlanke Bauweise erreicht wird, die zu einem erhöhten Tragekomfort des Behandlungsgerätes für den Patienten führt.

Die Teleskopfelemente 6 sind in ihrem Inneren mit einer Druckfeder 8 versehen, wie dies aus der Fig. 3 hervorgeht. Diese Druckfeder 8 kann bedarfsweise einfach ausgewechselt werden, so dass einfache und variable Einstellungen patientengerecht vorgenommen werden können, und zwar sowohl in der Protrusion als auch in der Wahl der wirkenden Kräfte. Im einzelnen sind die Teleskopelemente 6 von zwei Teleskophülsen 6.1 und einer Teleskopstange 6.2 gebildet.

Wie sich weiter aus der Fig. 3 ersehen läßt, ist die den Kugelkopf 7 tragende Teleskophülse 6.1 mit einer Innenteleskopschraube 9 versehen, der die Druckfeder 8 anliegt. Diese Innenteleskopschraube 9 ist über eine Bohrung 10 im Kugelkopf 7 einstellbar, so dass auf diese Weise eine weitere einfache Einstellmöglichkeit für die Federkraft gegeben ist. Dabei kann die Druckfeder 8 auch austauschbar in dem Teleskopelement 6 angeordnet sein.

Die Schienen 1, 2 bestehen vorteilhafterweise aus antiallergenem Kunststoff, wobei sich hierfür insbesondere das Spritzgußverfahren anbietet.

Im einzelnen können die Schienen 1, 2 aus einer Tiefziehfolie hergestellt sein, die aus einem Folienverbund aus Polyethylenterephthalat, Polyethylen und Polyurethan bestehen.

Die Schienen 1, 2 können aber ebenso aus einem Kaltpolymerisat oder aus Polymethylmethacrylat, insbesondere in Granulatform hergestellt sein, wobei das Material auf etwa 260° erhitzt und anschließend unter einem Druck von etwa 9 Bar in eine Küvettenform gepreßt wird.

Des weiteren sind die Teleskopelemente 6 im Hinblick auf eine erhöhte Biokompatibilität aus Titan gefertigt.

Wie sich schließlich aus der Fig. 4 ergibt, können die Kugelköpfe 7 in Matrizen 11 lagern, die in die Schienen 1, 2 einpolymerisiert sind. Auf diese Weise können optimale Lagerverhältnisse für die endseitig an den Teleskopelementen angebrachten Kugelköpfe 7 geschaffen werden.

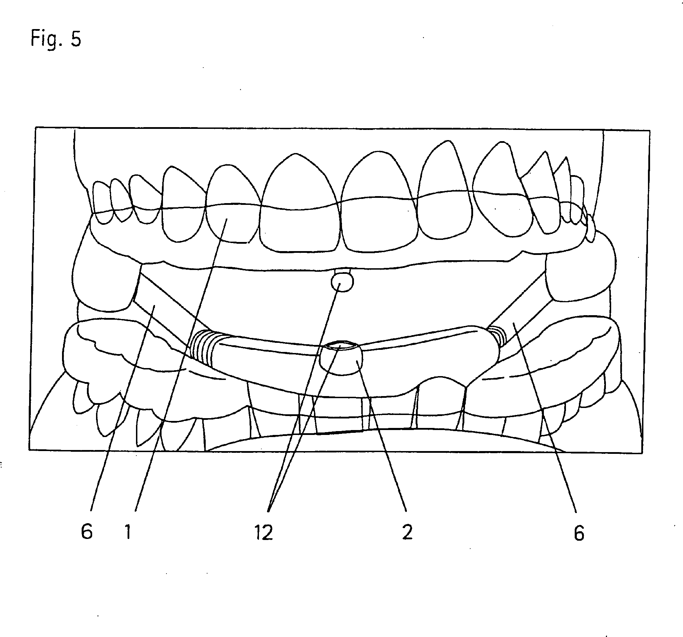

Schließlich kann gemäß Fig. 5 im frontalen Bereich der Schienen 1, 2 eine diese gegenseitig verriegelnde Verschlußeinrichtung 12 vorgesehen sein, wobei die Verschlußeinrichtung hier als Kugelknopfarretierung ausgebildet ist. Diese Kugelknopfarretierung kann in nicht näher wiedergegebener Weise als Rastsitz oder als eine lose Entlastungsposition ausgebildet sein.

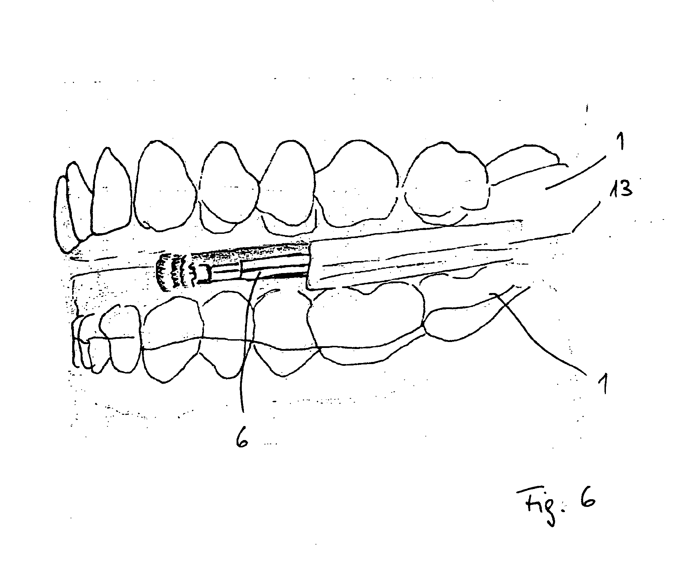

In Fig. 6 ist eine weitere Ausführungsmöglichkeit gezeigt, bei der auf einer der Schienen 1, 2 ein Bisswall 13 zur Abstützung und damit zur Entlastung der Muskulatur, Kiefer und Gelenke vorgesehen ist.

Diese Bisswälle 13 erstrecken sich - wie erkennbar - von okklusal nach buccal.

## Patentansprüche

1. Biokompatibles Behandlungsgerät zur Therapie des Schnarchens und schlafbezogener Atemstörungen von Patienten, bestehend aus einem Doppelschienensystem mit je einer Schiene (1, 2) für den Ober- und den Unterkiefer sowie einem Teleskopsystem (3), das von zwei Teleskopelementen (6) gebildet ist und mit seinen beiden Enden an jeweils einer der beiden Schienen (1, 2) gelenkig angeschlossen ist, **dadurch gekennzeichnet, daß** das Teleskopsystem (3) elastisch ist, daß die Teleskopelemente (6) jeweils vestibulär angeordnet sind, und endseitig jeweils mit einem Kugelkopf (7) versehen sind.

2. Behandlungsgerät nach Anspruch 1, **dadurch gekennzeichnet, daß** die Teleskopelemente (6) sich vom unteren Zahnbogen anterior zum oberen Zahnbogen posterior erstrecken.

3. Behandlungsgerät nach Anspruch 1 oder 2, **dadurch gekennzeichnet, daß** die Teleskopelemente (6) in ihrem Inneren mit einer Druckfeder (8) versehen sind.

4. Behandlungsgerät nach einem der Ansprüche 1 bis 3, **dadurch gekennzeichnet, daß** die Teleskopelemente (6) von zwei Teleskophülsen (6.1) und einer Teleskopstange (6.2) gebildet sind.

5. Behandlungsgerät nach Anspruch 4, **dadurch gekennzeichnet, daß** die den Kugelkopf (7) tragende Teleskophülse (6.1) mit einer Innenteleskopschraube (9) versehen ist, der die Druckfeder (8) anliegt und die über eine Bohrung (10) im Kugelkopf (7) einstellbar ist.

6. Behandlungsgerät nach Anspruch 4 oder 5, **dadurch gekennzeichnet, daß** die Druckfeder (8) austauschbar in dem Teleskopelement (6) angeordnet ist.

7. Behandlungsgerät nach einem der Ansprüche 1 bis 6, **dadurch gekennzeichnet, daß** die Schienen (1, 2) aus antiallergenem Kunststoff im Spritzgußverfahren hergestellt sind.

8. Behandlungsgerät nach einem der Ansprüche 1 bis 6, **dadurch gekennzeichnet, daß** die Schienen (1, 2) aus einer Tiefziehfolie hergestellt sind, die aus einem Folienverbund aus Polyethylenterephthalat, Polymethylen und Polyurethan bestehen.

9. Behandlungsgerät nach einem der Ansprüche 1 bis 6, **dadurch gekennzeichnet, daß** die Schienen (1, 2) aus einem Kaltpolymerisat hergestellt sind.

10. Behandlungsgerät nach einem der Ansprüche 1 bis 6, **dadurch gekennzeichnet, daß** die Schienen (1, 2) aus Polymethylmethacrylat, insbesondere in Granulatform hergestellt sind, wobei das Material auf etwa 260° erhitzt und anschließend unter einem Druck von etwa 9 Bar in eine Küvettenform gepreßt wird.

11. Behandlungsgerät nach einem der Ansprüche 1 bis 10, **dadurch gekennzeichnet, daß** die Teleskopelemente (6) aus Titan bestehen.

12. Behandlungsgerät nach einem der Ansprüche 1 bis 11, **dadurch gekennzeichnet, daß** die Kugelköpfe (7) in Matrizen lagern, die in die Schienen (1, 2) einpolymerisiert sind.

13. Behandlungsgerät nach einem der Ansprüche 1 bis 12, **dadurch gekennzeichnet, daß** im frontalen Bereich der Schienen (1, 2) eine diese gegenseitig verriegelnde Verschlußeinrichtung vorgesehen ist.

14. Behandlungsgerät nach Anspruch 13, **dadurch gekennzeichnet, daß** die Verschlußeinrichtung als Kugelknopfarretierung ausgebildet ist.

15. Behandlungsgerät nach Anspruch 14, **dadurch gekennzeichnet, daß** die Kugelknopfarretierung als Rastsitz oder als eine lose Entlastungsposition ausgebildet ist.

16. Behandlungsgerät nach einem der Ansprüche 1 bis 15, **dadurch gekennzeichnet, daß** auf wenigstens einer der Schienen (1, 2) Bisswälle (13) zur Abstützung und damit zur Entlastung der Muskulatur, Kiefer und Gelenke vorgesehen sind.

17. Behandlungsgerät nach Anspruch 16, **dadurch gekennzeichnet, daß** die Bisswälle (13) sich von okklusal nach buccal erstrecken.

## Claims

1. A biocompatible treatment device for therapy of snoring and sleep-related respiratory disturbances of patients, comprising a dual-rail system having a respective rail (1, 2) for the upper and lower jaws and a telescopic system (3) which is formed by two telescopic elements (6) and is hingedly connected with its two ends to a respective one of the two rails (1, 2), **characterised in that** the telescopic system (3) is elastic, the telescopic elements (6) are respectively arranged vestibularly and are each provided at their end with a ball head (7).

2. A treatment device according to claim 1 **characterised in that** the telescopic elements (6) extend from the lower dental arch anteriorly to the upper dental arch posteriorly.

3. A treatment device according to claim 1 or claim 2 **characterised in that** the telescopic elements (6) are provided with a compression spring (8) in their interior.

4. A treatment device according to one of claims 1 to 3 **characterised in that** the telescopic elements (6) are formed by two telescopic sleeves (6.1) and a telescopic bar (6.2).

5. A treatment device according to claim 4 **characterised in that** the telescopic sleeve (6.1) bearing the ball head (7) is provided with an internal telescopic screw (9) which bears against the compression spring (8) and which is adjustable by way of a bore (10) in the ball head (7).

6. A treatment device according to claim 4 or claim 5 **characterised in that** the compression spring (8) is arranged interchangeably in the telescopic element (6).

7. A treatment device according to one of claims 1 to 6 **characterised in that** the rails (1, 2) are made from anti-allergic plastic material in an injection moulding process.

8. A treatment device according to one of claims 1 to 6 **characterised in that** the rails (1, 2) are made from a deep-drawing film comprising a composite film of polyethylene terephthalate, polyethylene and polyurethane.

9. A treatment device according to one of claims 1 to 6 **characterised in that** the rails (1, 2) are made from a cold polymer.

10. A treatment device according to one of claims 1 to 6 **characterised in that** the rails (1, 2) are made from polymethyl methacrylate, in particular in granule form, wherein the material is heated to about 260° and then pressed under a pressure of about 9 bars into a tray shape.

11. A treatment device according to one of claims 1 to 10 **characterised in that** the telescopic elements (6) comprise titanium.

12. A treatment device according to one of claims 1 to 11 **characterised in that** the ball heads (7) are mounted in female members which are polymerised into the rails (1, 2).

13. A treatment device according to one of claims 1 to 12 **characterised in that** in the frontal region of the rails (1, 2) there is provided a closure device for mutually locking same.

14. A treatment device according to claim 13 **characterised in that** the closure device is in the form of a ball knob arresting means.

15. A treatment device according to claim 14 **characterised in that** the ball knob arresting means is in the form of a latching seat or in the form of a loose release position.

16. A treatment device according to one of claims 1 to 15 **characterised in that** provided on at least one of the rails (1, 2) are bite blocks (13) for supporting and thus for relieving the load on the muscles, jaw and joints.

17. A treatment device according to claim 16 **characterised in that** the bite blocks (13) extend from occlusal towards buccal.

## Revendications

1. Appareil de traitement biocompatible destiné au traitement du ronflement et des gênes respiratoires liées au sommeil de patients, composé d'un système de double gouttière, comportant respectivement une gouttière (1, 2) pour la mâchoire supérieure et la mâchoire inférieure, ainsi que d'un système télescopique (3) qui est formé de deux éléments télescopiques (6) et est articulé avec ses deux extrémités respectivement sur l'une des deux gouttières (1, 2), **caractérisé par le fait que** le système télescopique (3) est élastique et que les éléments télescopiques (6) sont chacun disposés de façon vestibulaire et sont pourvus d'une tête sphérique (7) à leurs extrémités.

2. Appareil de traitement selon la revendication 1, **caractérisé par le fait que** les éléments télescopiques (6) s'étendent de l'arcade dentaire inférieure antérieure à l'arcade dentaire supérieure postérieure.

3. Appareil de traitement selon la revendication 1 ou 2, **caractérisé par le fait que** les éléments télescopiques (6) sont pourvus à l'intérieur d'un ressort de pression (8).

4. Appareil de traitement selon une des revendications 1 à 3, **caractérisé par le fait que** les éléments télescopiques (6) sont constitués de deux tubes télescopiques (6.1) et d'une tige télescopique (6.2).

5. Appareil de traitement selon la revendication 4, **caractérisé par le fait que** le tube télescopique (6.1) portant la tête sphérique (7) est pourvu d'une vis télescopique interne (9) contre laquelle s'appuie le ressort de pression (8) et qui peut être réglée en passant par un trou (10) de la tête sphérique (7).

6. Appareil de traitement selon la revendication 4 ou 5, **caractérisé par le fait que** le ressort de pression (8) est disposé de manière échangeable dans l'élément télescopique (6).

7. Appareil de traitement selon une des revendications 1 à 6, **caractérisé par le fait que** les gouttières (1,2) sont fabriquées à partir d'une matière synthétique antiallergique, par un procédé de moulage par injection.

8. Appareil de traitement selon une des revendications 1 à 6, **caractérisé par le fait que** les gouttières (1, 2) sont fabriquées à partir d'une feuille d'emboutissage qui est constituée d'une feuille composite en polyéthylène téréphtalate, polyéthylène et polyuréthane.

9. Appareil de traitement selon une des revendications 1 à 6, **caractérisé par le fait que** les gouttières (1, 2) sont fabriquées à partir d'un produit de polymérisation à froid.

10. Appareil de traitement selon une des revendications 1 à 6, **caractérisé par le fait que** les gouttières (1, 2) sont fabriquées à partir de polyméthacrylate de méthyle, en particulier sous forme de granulés, la matière étant chauffée à environ 260° et pressée ensuite avec une pression d'environ 9 bars dans un moule à cuvette.

11. Appareil de traitement selon une des revendications 1 à 10, **caractérisé par le fait que** les éléments télescopiques (6) sont en titane.

12. Appareil de traitement selon une des revendications 1 à 11, **caractérisé par le fait que** les têtes sphériques (7) sont montées dans des matrices qui sont intégrées par polymérisation dans les gouttières (1, 2).

13. Appareil de traitement selon une des revendications 1 à 12, **caractérisé par le fait que** dans la partie frontale des gouttières (1, 2), il est prévu un dispositif de fermeture destiné à les verrouiller mutuellement.

14. Appareil de traitement selon la revendication 13, **caractérisé par le fait que** le dispositif de fermeture est réalisé sous forme de moyen de verrouillage à bouton sphérique.

15. Appareil de traitement selon la revendication 14, **caractérisé par le fait que** le moyen de verrouillage à bouton sphérique est réalisé sous forme d'élément d'encliquetage ou comme position de décharge mobile.

16. Appareil de traitement selon une des revendications 1 à 15, **caractérisé par le fait qu'**il est prévu sur au moins une des gouttières (1, 2), des bourrelets (13) destinés à soutenir et donc à soulager les muscles, les mâchoires et les articulations.

17. Appareil de traitement selon la revendication 16, **caractérisé par le fait que** les bourrelets (13) s'étendent du côté occlusal vers le côté buccal.
